(19) Europäisches Patentamt
European Patent Office
Office européen des brevets

(11) Veröffentlichungsnummer: **0 306 938 A2**

(12) # EUROPÄISCHE PATENTANMELDUNG

(21) Anmeldenummer: **88114677.3**

(51) Int. Cl.[4]: **C07D 457/02 , A61K 31/48**

(22) Anmeldetag: **08.09.88**

(30) Priorität: **08.09.87 DE 3730124**

(43) Veröffentlichungstag der Anmeldung: **15.03.89 Patentblatt 89/11**

(84) Benannte Vertragsstaaten: **CH DE FR GB IT LI NL**

(71) Anmelder: **Eich, Eckart, Prof. Dr.**
**Dietrich Schäfer-Weg 16-18**
**D-1000 Berlin 41(DE)**

(72) Erfinder: **Eich, Eckart, Prof. Dr.**
**Dietrich-Schäfere-Weg 16-18**
**D-1000 Berlin 41(DE)**
Erfinder: **Pertz, Heinz, Dr.**
**Grunewaldstrasse**
**D-1000 Berlin 41(DE)**
Erfinder: **Müller, Werner E. G., Prof. Dr.**
**Semmelweisstrasse 12**
**D-6200 Wiesbaden-Biebrich(DE)**

(74) Vertreter: **Kraus, Walter, Dr. et al**
**Patentanwälte Kraus, Weisert & Partner**
**Thomas-Wimmer-Ring 15**
**D-8000 München 22(DE)**

(54) **Festuclavin- und Pyroclavin-Derivate, Verfahren zu ihrer Herstellung und diese Verbindungen enthaltende Arzneimittel.**

(57) Beschrieben werden neue Festuclavin- und Pyroclavinderivate der allgemeinen Formel I

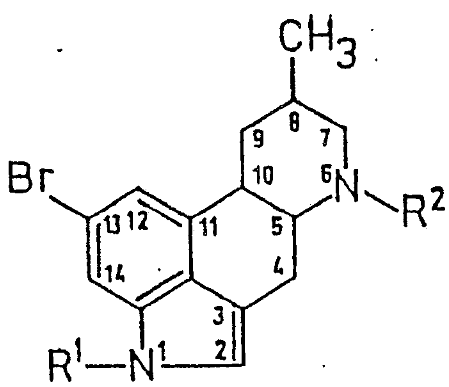

(I)

sowie ihre physiologisch annehmbaren Salze. Die neuen Verbindungen zeichnen sich durch wertvolle pharmakologische Eigenschaften und insbesondere durch eine hohe antineoplastische Wirksamkeit aus.

EP 0 306 938 A2

Xerox Copy Centre

# Festuclavin- und Pyroclavin-Derivate, Verfahren zu ihrer Herstellung und diese Verbindungen enthaltende Arzneimittel

Die Erfindung betrifft neue Festuclavin- und Pyroclavin-Derivate sowie deren physiologisch annehmbare Salze. Die Erfindung betrifft weiterhin ein Verfahren zur Herstellung dieser Derivate und diese Verbindungen enthaltende Arzneimittel bzw. die Verwendung dieser Derivate zur Herstellung von Azneimitteln zur Bekämpfung von Tumoren.

Die beiden 8-epimeren Mutterkornalkaloide Festuclavin (Methylgruppe an C-8 β-ständig) und Pyroclavin (Methylgruppe an C-8 α-ständig) sind aus J. agric. chem. Soc. (Japan), 28, 501 (1954) bzw. Bull. agric. chem. Soc. (Japan), 20, 59 (1956) bekannt.

Es wurde nun gefunden, daß bestimmte Festuclavin- und Pyroclavin-Derivate sowie die physiologisch annehmbaren Salze davon wertvolle pharmakologische Eigenschaften besitzen und sich vor allem durch eine hohe antineoplastische Wirksamkeit auszeichnen.

Gegenstand der Erfindung sind daher Festuclavin- und Pyroclavin-Derivate der allgemeinen Formel I

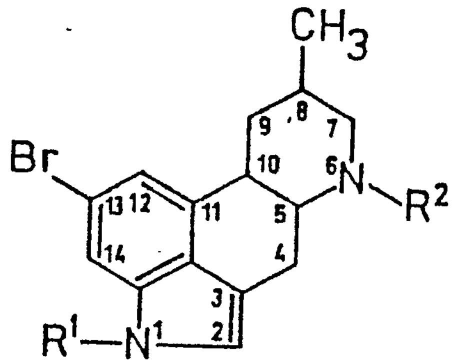

(I)

worin $R^1$ für $C_{3-5}$-Cycloalkyl, $C_{3-5}$-Cycloalkylmethyl, Methyl-$C_{3-5}$-cycloalkyl oder Methyl-$C_{3-5}$-cycloalkylmethyl steht und $R^2$ für geradkettiges oder verzweigtes $C_{1-6}$-Alkyl, $C_{3-5}$-Cycloalkyl, $C_{3-5}$-Cycloalkylmethyl, Methyl-$C_{3-5}$-cycloalkyl oder Methyl-$C_{3-5}$-cycloalkylmethyl steht, sowie ihre physiologisch annehmbaren Salze.

In der allgemeinen Formel I steht $R^1$ für eine $C_{3-5}$-Cycloalkylgruppe, beispielsweise die Cyclopropyl-, Cyclobutyl- oder Cyclopentylgruppe, oder eine $C_{3-5}$-Cycloalkylmethylgruppe, wie eine Cyclopropylmethyl-, Cyclobutylmethyl- oder Cyclopentylmethylgruppe. Weiterhin kann $R^1$ auch für eine Methyl-$C_{3-5}$-cycloalkyl-, beispielsweise eine Methylcyclopropyl-, 2-Methylcyclobutyl- oder 3-Methylcyclobutylgruppe, stehen. Schließlich kann $R^1$ auch eine Methyl-$C_{3-5}$-cycloalkylmethylgruppe, beispielsweise eine Methylcyclopropylmethyl-, 2-Methylcyclobutylmethyl- oder 3-Methylcyclobutylmethylgruppe bedeuten.

In der allgemeinen Formel I steht weiterhin $R^2$ für geradkettiges oder verzweigtes $C_{1-6}$-Alkyl, wie Methyl, Ethyl, n- und Isopropyl, n- und Isobutyl und n- und Isopentyl, $C_{3-5}$-cycloalkyl, $C_{3-5}$-cycloalkylmethyl, Methyl-$C_{3-5}$-cycloalkyl oder Methyl-$C_{3-5}$-cycloalkylmethyl. Beispiele für diese Gruppen sind die oben im Zusammenhang mit $R^1$ angegebenen Gruppen.

Bei den physiologisch annehmbaren Salzen der Festuclavin- und Pyroclavin-Derivate der allgemeinen Formel I handelt es sich um die Umsetzungsprodukte mit geeignten Säuren. Diese Salze können zum Beispiel mit Mineralsäuren, wie Chlor-, Brom- oder lodwasserstoffsäure, Phosphorsäure, Salpetersäure oder Schwefelsäure, oder mit organischen Säuren, wie Ameisensäure, Essigsäure, Propionsäure, Weinsäure, Zitronensäure, Maleinsäure, Fumarsäure, Methansulfonsäure, Embonsäure etc., in bekannter Weise gebildet werden. Die Erfindung umfaßt daher auch alle physiologisch annehmbaren Salze der oben bechriebenen Derivate der allgemeinen Formel I.

Die Erfindung umfaßt weiterhin auch alle stereoisomeren sowie alle tautomeren Formen und Hydrate der oben beschriebenen Verbindungen der allgemeinen Formel I.

Die erfindungsgemäßen Verbindungen können durch ein Verfahren hergestellt werden, das dadurch

gekennzeichnet ist, daß man

(a) eine Verbindung der allgemeinen Formel II

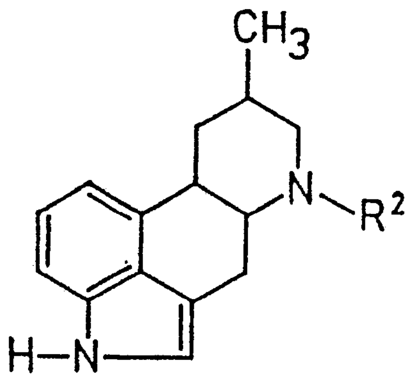

(II)

worin $R^2$ die oben angegebene Bedeutung hat, mit elementarem Brom zum entsprechenden 2,13-Dibromderivat III

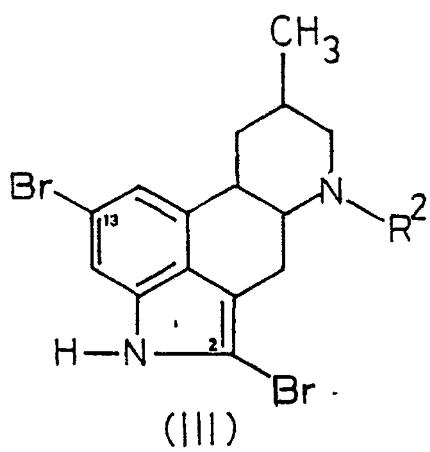

(III)

umsetzt,

(b) das erhaltene Derivat III zum 13-Monobromderivat IV

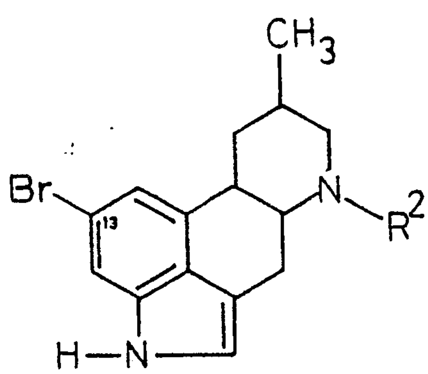

(IV)

reduziert,

(d) das erhaltene Derivat IV mit einer Verbindung der allgemeinen Formel V

$R^1Hal$ (V)

worin $R^1$ die oben angegebene Bedeutung hat und Hal die Bedeutung Chlor, Brom oder Iod hat, umsetzt und daß man

(d) die so erhaltene Verbindung gegebenenfalls in ihr physiologisch annehmbares Salz umwandelt.

Die Ausgangsstoffe für das erfindungsgemäße Verfahren können, soweit sie nicht bekannt sind, in Analogie zu bekannten Methoden hergestellt werden (E. Eich et al., Arch. Pharm. (Weinheim) 318, 214 (1985), E. Eich et al., J. Antibiot, 39, 804 (1986).

Im einzelnen umfaßt das erfindungsgemäße Verfahren zur Herstellung der oben definierten Derivate I folgende Stufen:

(a) In der ersten Stufe wird eine Verbindung der allgemeinen Formel II, worin $R^2$ die oben angegebene Bedeutung hat, mit elementarem Brom zum entsprechenden 2,13-Dibromderivat III umgesetzt. Die Umsetzung erfolgt in Eisessig als Medium und einem Molverhältnis von Derivat II zu Brom von vorzugsweise 1:2,2. Die anfängliche Reaktionstemperatur beträgt 5 bis 15 °C vorzugsweise 10 °C. Das Brom kann als elementares Brom oder vorzugsweise in Form seiner Additionsverbindung Pyridiniumhydrobromid-perbromid eingesetzt werden. Die Isolierung und Reinigung des Zwischenprodukts III erfolgt in üblicher Weise, beispielsweise durch Basischstellen, Abtrennen des daraufhin gebildeten Niederschlages, Wiederauflösen in ammoniakalischem Methanol und Einengen bis zur beginnenden Kristallisation.

Die Ausgangsverbindungen der Formel II umfassen Festuclavin und Pyroclavin sowie deren N-6-homolog substituierte Derivate mit 2 bis 6 C-Atomen, die geradkettig (zum Beispiel 6-Ethyl-6-norfestuclavin) oder verzweigt angeordnet sein oder Cycloalkyl sowie Cycloalkylmethyl (zum Beispiel 6-Cyclopropylmethyl-6-norfestuclavin) darstellen können.

(b) In der zweiten Stufe wird das erhaltene Derivat III zum 13-Monobromderivat IV reduziert, was vorzugsweise mit Natriumborhydrid und in Gegenwart von Kobaltsalzen geschieht. Diese Stufe wird gemäß der DE-OS 2 330 912 durchgeführt. Die Reduktion erfolgt in Methanol/THF als Reaktionsmedium und bei Temperaturen von -15 bis -25 °C, vorzugsweise -20 °C. Die Isolierung und Reinigung des Zwischenprodukts IV erfolgt auf übliche Weise, beispielsweise - nach geeigneter Extraktion und Konzentrierung - durch zentrifugale präparative Dünnschichtchromatographie.

(c) In der dritten Stufe des erfindungsgemäßen Verfahrens wird das Derivat IV mit einer Verbindung der allgemeinen Formel V umgesetzt. Als Verbindungen der Formel $R^1Hal$ kommen beispielsweise in Frage Cycloalkylhalogenide, wie Cyclopropyl-, Cyclobutyl- und Cyclopentylbromid; Cycloalkylmethylhalogenide, wie Cyclopropylmethyl-, Cyclobutylmethyl- und Cyclopentylmethyliodid; Methylcycloalkylhalogenide, wie Methylcyclopropyl-, 2-Methylcyclobutyl- und 3-Methylcyclobutylbromid; Methylcycloalkylmethylhalogenide, wie Methylcyclopropylme thyl-, 2-Methylcyclobutylmethyl- und 3-Methylcyclobutylmethyliodid.

Die in dieser Stufe erfolgende Substitution an N-1 kann dadurch erfolgen, daß man die Reaktanten in flüssigem Ammoniak/Kaliumamid zur Umsetzung bringt, wie es von F. Troxler und A. Hofmann in Helv. Chim. Acta 40, 1706 (1957) beschrieben wird. Die Umsetzung erfolgt in diesem Falle bei Reaktionstemperaturen von -40 bis -50 °C, vorzugsweise -45 °C, und unter Anwendung von Molverhältnissen von vorzugsweise 1:1,4.

Die Umsetzung in dieser Stufe kann aber - was bevorzugt wird - auch in Tetrahydrofuran oder anderen geeigneten Lösungsmitteln, wie Dichlormethan, und Aceton/Kaliumhydroxid/Tetraalkylammoniumsalzen (zum Beispiel Tetra-n-butyl-ammoniumhydrogensulfat) erfolgen, wie es in der DE-OS 3 309 493 beschrieben wird. In diesem Falle kommen Reaktionstemperaturen von 10 bis 30 °C, vorzugsweise 20 °C, und Molverhältnissen von vorzugsweise 1:5 zur Anwendung.

Die Isolierung und Reinigung erfolgt in üblicher Weise, beispielsweise - nach geeigneter Extraktion und Konzentrierung - durch zentrifugale präparative Dünnschichtchromatographie.

In der letzten Stufe des erfindungsgemäßen Verfahrens werden die erhaltenen Produkte gegebenenfalls in an sich bekannter Weise in ihre physiologisch annehmbaren Salze umgewandelt, wozu beispielsweise die vorstehend genannten Säuren verwendet werden können.

Die erfindungsgemäßen Verbindungen können auch in umgekehrter Reihenfolge, d.h. zuerst N-1-Alkylierung und anschließend Bromierung, gewonnen werden, jedoch mit schlechteren Gesamtausbeuten.

Wie bereits oben ausgeführt, zeichnen sich die erfindungsgemäßen Derivate durch eine hohe antineoplastische Wirksamkeit aus, was an verschiedenen Krebszell-Linien tierischer und menschlicher Herkunft in vitro belegt werden konnte. Es ist zwar bekannt, daß 1-substituierte Clavine und deren an N-6 variierte Derivate ausgeprägte cytostatische Eigenschaften besitzen (DE-OS 34 03 067). Gegenüber den Verbindungen dieser Druckschrift zeichnen sich die erfindungsgemäßen Verbindungen jedoch durch stark verbesserte metabolische Stabilität aus, so daß sie ausgezeichnete, in vivo den Verbindungen der DE-OS 34 03 067 überlegene Wirksamkeit beim L5178y-Mäuselymphom zeigen. So ergab zum Beispiel 13-Brom-1-cyclopropylmethyl-festuclavin bei Tumormäusen unter 5tägiger Applikation von 50 mg/kg einen T/C-Wert von 212 %. Angesichts ihrer geringen akuten Toxizität und ihrer hohen Wirksamkeit ist eine Verwendung bei der Chemotherapie von Krebserkrankungen zu erwarten.

Die erfindungsgemäßen Verbindungen können zur Verabreichung in jeder beliebigen Weise formuliert werden. Die Erfindung umfaßt daher auch Arzneimittel, die mindestens eine erfindungsgemäße Verbindung zur Verwendung in der Human- oder Veterinärmedizin enthalten. Solche Arzneimittel können herkömmlicherweise unter Verwendung von einem oder mehreren pharmazeutischen Trägern oder Verdünnungsmitteln hergestellt werden.

Die erfindungsgemäßen Verbindungen könner daher für die orale, bukkale, topische, parenterale oder rektale Verabreichung formuliert werden. Für die orale Verabreichung kann das Arzneimittel in Form von beispielsweise Tabletten, Kapseln, Pulvern, Lösungen, Sirups oder Suspensionen vorliegen, die unter Verwendung von annehmbaren Verdünnungsmitteln auf herkömmliche Weise hergestellt worden sind. Für die bukkale Verabreichung kann das Arzneimittel die Form von Tabletten oder Briefchen einnehmen, die in herkömmlicher Weise formuliert worden sind.

Die erfindungsgemäßen Verbindungen können für die parenterale Verabreichung durch Bolusinjektion oder kontinuierliche Infusion formuliert werden. Formulierungen zur Injektion können in Dosiseinheitsform als Ampullen oder in Mehrfachdosenbehältern mit zugesetztem Konservierungsmittel vorliegen.

Die Arzneimittel können solche Formen, wie Suspensionen, Lösungen oder Emulsionen, in öligen oder wäßrigen Trägern einnehmen, und sie können Formulierungshilfsmittel, wie Suspendierungs-, Stabilisierungs- und/oder Dispergierungsmittel, enthalten.

Alternativ kann der Wirkstoff auch in Pulverform zur Rekonstitution mit einem geeigneten Träger, zum Beispiel sterilem, pyrogrenfreiem Wasser, vor dem Gebrauch vorliegen.

Die erfindungsgemäßen Verbindungen können auch für rektale Zubereitungen, zum Beispiel Suppositorien oder Retentionseinläufe, formuliert werden, die herkömmliche Suppositoriengrundlagen, wie Kakaobutter oder andere Glyceride, enthalten.

Zur topischen Anwendung können die erfindungsgemäßen Verbindungen als Salben, Cremes, Gels, Lotionen, Pulver oder Sprays in herkömmlicher Weise formuliert werden.

Die Beispiele erläutern die Erfindung.

## Beispiel 1

(13-Brom-1-cyclopropylmethyl-festuclavin-hydrogenmaleat)

1 g (4,16 mMol) Festuclavin werden in 30 ml Essigsäure gelöst, die Lösung auf 10° C abgekühlt und mit 2,93 g (9,16 mMol) Pyridinhydrobromidperbromid behandelt. Nach 2stündigem Rühren bei Raumtemperatur wird das Lösungsmittel teilweise abdestilliert. Der Rückstand wird im Eisbad gekühlt und mit konz. Ammoniakflüssigkeit basisch gestellt. Das Präzipitat wird abfiltriert und in einer warmen Lösung von konz. Ammoniakflüssigkeit in Methanol (8:100) gelöst. Nach Behandlung der Lösung mit Aktivkohle wird filtriert und bis zum Beginn der Kristallisation von 2,13-Dibrom-festuclavin eingedampft. Ausbeute: 40 %; Schmelzpunkt 300° C.

1,2 g (3 mMol) 2,13-Dibrom-festuclavin werden in 170 ml Methanol-Tetrahydrofuran (3:2) gelöst und anschließend bei -20° C tropfenweise und unter Rühren wäßrige Lösungen von $CoCl_2$ x $H_2$) (3,8 g/10 ml) und Natriumborhydrid (3,0 g/10 ml) hinzugefügt. Die Mischung wird weitere 30 Minuten bei -20° C gerührt, anschließend mit Wasser verdünnt und mit Ethylacetat extrahiert. Das Lösungsmittel wird abgedampft und der Rückstand mittels zentrifugaler präparativer Dünnschichtchromatographie (Sorbens: Kieselgel $60PF_{254}$ Merck; Laufmittel: Dichlormethan/Methanol 90:10) aufgetrennt. Hierdurch gelingt die Abtrennung von 13-Brom-festuclavin. Ausbeute: 40 %; Schmelzpunkt: 200° C.

Zu einer Lösung von 160 mg (0,5 mMol) 13-Brom-festuclavin in 10 ml Tetrahydrofuran werden bei Raumtemperatur 250 mg (4,5 mMol) Kaliumhydroxid, 17 mg (0,05 mMol) Tetra-n-butylammoniumhydrogensulfat und 340 mg (2,5 mMol) Cyclopropylmethylbromid gegeben. Die Mischung wird 3 Stunden gerührt. Das Präzipitat wird abfiltriert und mit Dichlormethan gewaschen. Dann wird das Lösungsmittel abgedampft und der Rückstand zwischen Dichlormethan und einer gesättigten wäßrigen Natriumbikarbonat-Lösung verteilt. Die organische Phase wird abgetrennt, mit Natriumsulfat getrocknet und anschließend zur Trockne eingedampft. Aus dem Rückstand wird mittels zentrifugaler präparativer Dünnschichtchromatographie (Sorbens: wie oben; Laufmittel: Dichlormethan/Methanol 95:5) 13-Brom-1-cyclopropylmethyl-festuclavin isoliert und mit Maleinsäure als Hydrogenmaleat aus Tetrahydrofuran/Diethylether kristallisiert. Ausbeute: 75 %; Schmelzpunkt: 168° C. NMR-Daten:

$^1$H-NMR ($CDCl_3$) 0,34 und 0,61

$$(2m,\ 4H,\ N\text{-}CH_2\text{-}CH\text{-}\underline{CH_2}\text{-}\underline{CH_2});$$

1,10 (d, J = 6 Hz, 3H); 1,15-1,23

$$(m,\ 1H,\ N\text{-}CH_2\text{-}\underline{CH}\text{-}CH_2\text{-}CH_2);$$

1.27 (q, teilweise überlagert, J = 12Hz, 1H); 2,3-2,55 (m, 2H); 2,71 (pseudo d, 1H); 2,97 (ca. s, 3H, und 1H überlagert); 3,25 (m, 1H); 3,4-3.65 (m, 3H); 3,88 (d J = 7Hz, 2H, N-$\underline{CH_2}$-$C_3H_5$); 6,24 (s, 2H); 6,91 (s, 1H, H-C-(2)); 7,01 (s, 1H, H-C(12)); 7,35 (s, 1H, H-C(14)).

## Beispiel 2

(13)-Brom-1-cyclobutylmethyl-festuclavin-hydrogenmaleat)

In analoger Weise zu Beispiel 1 wird aus Festuclavin mit Hilfe von Cyclobutylmethylbromid das 13-Brom-1-cyclobutylmethyl-festuclavinhydrogenmaleat erhalten. Ausbeute: 65 % (bezogen auf die letzte Stufe).

## Beispiel 3

(13-Brom-1-cyclobutyl-festuclavin-hydrogenmaleat)

In analoger Weise zu Beispiel 1 wird aus Festuclavin mit Hilfe von Cyclobutylbromid das 13-Brom-1-cyclobutyl-festuclavin-hydrogenmaleat erhalten. Ausbeute: 55 % (bezogen auf die letzte Stufe).

## Beispiel 4

(13-Brom-1-cyclopropylmethyl-6-ethyl-6-norfestuclavin-hydrogenmaleat)

In analoger Weise zu Beispiel 1 wird aus 6-Ethyl-6-norfestuclavin mit Hilfe von Cyclopropylmethylbromid das 13-Brom-1-cyclopropylmethyl-6-ethyl-6-norfestuclavin-hydrogenmaleat erhalten. Ausbeute: 35 % (bezogen auf die letzte Stufe).

Beispiel 5

(13-Brom-1-cyclopropylmethyl-pyroclavin-hydrogenmaleat)

In analoger Weise zu Beispiel 1 wird aus Pyroclavin mit Hilfe von Cyclopropylmethylbromid das 13-Brom-1-cyclopropylmethyl-pyroclavinhydrogenmaleat erhalten. Ausbeute: 35 % (bezogen auf die letzte Stufe).

Beispiel 6

(13-Brom-1,6-dicyclopropylmethyl-6-norpyroclavin-hydrogenmaleat)

In analoger Weise zu Beispiel 1 wird aus 6-Cyclopropylmethyl-6-norpyroclavin mit Hilfe von Cyclopropylmethylbromid das 13-Brom-1,6-dicyclopropylmethyl-6-norpyroclavin-hydrogenmaleat erhalten. Ausbeute: 40 % (bezogen auf die letzte Stufe).

## Ansprüche

1. Festuclavin- und Pyroclavin-Derivate der allgemeinen Formel I

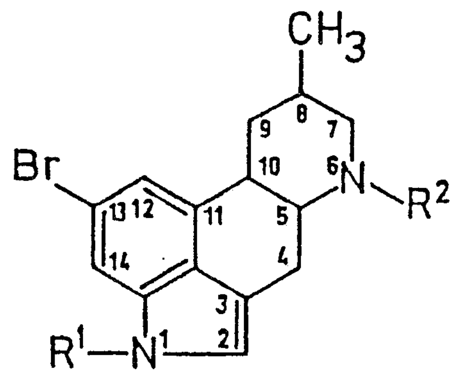

(I)

worin $R^1$ für $C_{3-5}$-Cycloalkyl, $C_{3-5}$-Cycloalkylmethyl, Methyl-$C_{3-5}$-cycloalkyl oder Methyl-$C_{3-5}$-cycloalkylmethyl steht; und
$R^2$ für geradkettiges oder verzweigtes $C_{1-6}$-Alkyl, $C_{3-5}$-Cycloalkyl, $C_{3-5}$-Cycloalkylmethyl, Methyl-$C_{3-5}$-cycloalkyl oder Methyl-$C_{3-5}$-cycloalkylmethyl steht,
sowie ihre physiologisch annehmbaren Salze.

2. Verfahren zur Herstellung von Festuclavin- und Pyroclavin-Derivaten nach Anspruch 1 sowie der physiologisch annehmbaren Salze davon, dadurch **gekennzeichnet,** daß man

(a) eine Verbindung der allgemeinen Formel II

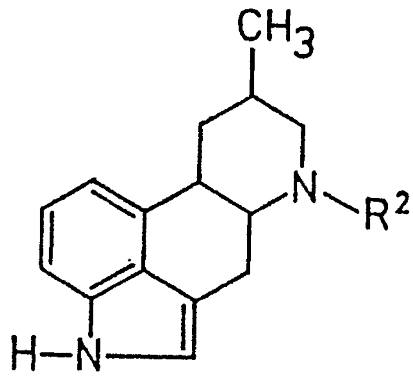

(II)

worin $R^2$ die in Anspruch 1 angegebene Bedeutung hat, mit elementarem Brom zum entsprechenden 2,13-Dibromderivat III

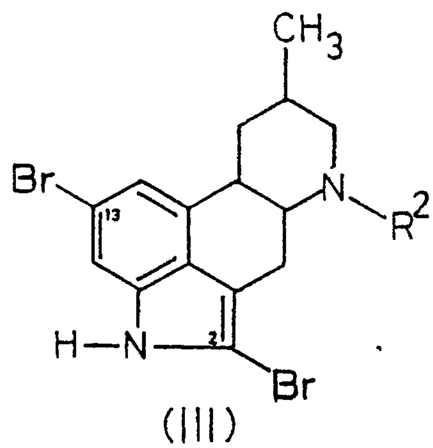

(III)

umsetzt,

(b) das erhaltene Derivat III zum 13-Monobromderivat IV

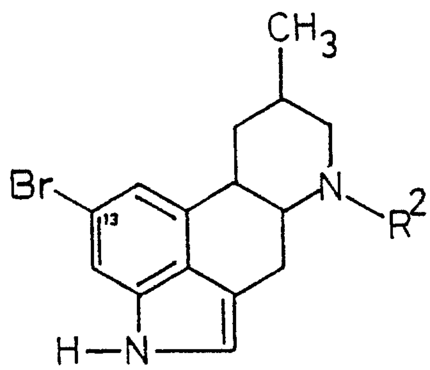

(IV)

reduziert,

(c) das erhaltene Derivat IV mit einer Verbindung der allgemeinen Formel V

$R^1Hal$ (V)

worin $R^1$ die in Anspruch 1 angegebene Bedeutung hat und Hal die Bedeutung Chlor, Brom oder Iod hat, umsetzt und daß man

(d) die so erhaltene Verbindung gegebenenfalls in ihr physiologisch annehmbares Salz umwandelt.

3. Arzneimittel, dadurch **gekennzeichnet,** daß es neben üblichen Hilfs- und Trägerstoffen wenigstens ein Festuclavin- oder Pyroclavin-Derivat der allgemeinen Formel I oder ein physiologisch annehmbares Salz davon nach Anspruch 1 enthält.

4. Verwendung eines Festuclavin- oder Pyroclavin-Derivats der allgemeinen Formel I sowie der physiologisch annehmbaren Salze davon nach Anspruch 1 zur Herstellung eines Arzneimittels zur Bekämpfung von Tumoren.